Europäisches Patentamt

European Patent Office

Office européen des brevets

(19)

(11) Publication number: **0 037 020**
**B1**

(12) **EUROPEAN PATENT SPECIFICATION**

(45) Date of publication of patent specification: **19.12.84**

(51) Int. Cl.³: **C 07 D 307/60,** B 01 J 35/02,
B 01 J 27/18, B 01 J 23/28

(21) Application number: **81102066.8**

(22) Date of filing: **19.03.81**

(54) **Catalyst for the preparation of maleic anhydride.**

(30) Priority: **02.04.80 IT 2113580**

(43) Date of publication of application:
**07.10.81 Bulletin 81/40**

(45) Publication of the grant of the patent:
**19.12.84 Bulletin 84/51**

(84) Designated Contracting States:
**AT CH DE FR GB LI**

(56) References cited:
**FR-A-1 262 809**
**FR-A-2 171 431**
**FR-A-2 303 594**
**GB-A- 893 987**
**GB-A-1 373 351**
**US-A-3 848 033**

**Handbook of Processes and Proven Catalysts**

**Handbook of Catalyst Manufacture, Sittig,**
**1978**

(73) Proprietor: **ALUSUISSE ITALIA S.p.A.**
**Via Vittor Pisani, 31**
**I-20124 Milano (IT)**

(72) Inventor: **Di Cio', Alessandro**
**Via Pignolo, 65**
**I-24100 Bergamo (IT)**
Inventor: **Vitali, Angelo**
**Via Marconi, 21**
**I-24020 Scanzorosciate (IT)**

(74) Representative: **Modiano, Guido et al**
**MODIANO & ASSOCIATI S.A.S. Via Meravigli, 16**
**I-20123 Milan (IT)**

Courier Press, Leamington Spa, England.

# 0 037 020

## Description

This invention relates in general to the preparation of maleic anhydride by oxidation of benzene (benzol), and more particularly to a catalyst useful in the preparation of maleic anhydride.

In conventional processes for the preparation of maleic anhydride by oxidation of benzene in vapor phase with an oxygen-containing gas, supported catalysts are currently employed which comprise in most cases, as the active portion thereof, various combinations of metal oxides, such as vanadium-molybdenum, tin-vanadium, iron-chromium, chromium-vanadium, etc., with minor additions of some auxiliary compounds, and as the carrier portion thereof, non-reactive particulate material, notably silica or alumina, which provide the required surface area, strength, and stability of the catalyst.

The maleic anhydride catalysts used heretofore, comprised supports or carriers of either irregular shape, or preferably, shaped as balls or cylinders.

While such known catalysts exhibit a satisfactory catalytic activity in the preparation of maleic anhydride, they are not entirely devoid of shortcomings, as connected with their potentiality and selectivity levels, as well as their capabilities to perform in mixtures at relatively high benzene concentrations.

For example GB—A—1 373 351 discloses variously shaped catalysts for vapour phase reactions. These are homogeneous type catalysts where the active mass is homogeneized with a binder and is contained not as a surface coating but is distributed throughout the entire mass of the catalyst. The various illustrated shapes do not include half-ring shaped catalyst.

GB—A—1 547 338 and US—A—3 848 033 discloses catalyst for the preparation of phthalic anhydride which are in the form of an amphora, i.e. a sphere with a central hole communicating with a surface aperture. Also these catalysts are of homogeneous type.

"Handbook of Catalyst Manufacture," Sitting, Noyes Data Corpn. 1978 shows catalysts for preparing maleic anhydride by oxidation of benzene by using as catalyst components 1 part $MoO_3$, 1.2 to 2.5 parts $V_2O_5$, 0.2—1.1 parts $Bi_2O_3$, 0.02 to 0.05 parts $P_2O_5$, with minor amounts of $Na_2O$, $SiO_2$. Such catalysts are used on supports of aggregate pellets form.

"Catalytic Processes and Proven Catalysts", Thomas, Academic Press 1970, pages 203—205 discloses the analogy between the catalytic oxidation processes for the preparation of phthalic anhydride and maleic anhydride.

GB—A—893 987 discloses a pellet shaped unsupported catalyst for decomposing hydrogen peroxide.

Finally, FR—A—2 303 594 discloses a catalyst useful, inter alia, for oxidizing benzene to maleic anhydride which comprises a vanadium and titanium based active mass coated on a non-porous ringshaped support.

It is a principal object of the present invention to provide a catalyst for use in the preparation of maleic anhydride, which has improved properties over conventional catalysts as relates to its performances and in particular its potentiality and selectivity, even where benzene/air mixtures of higher than normal concentrations are used.

It is another object of the present invention, to provide a catalyst which has a higher activity or durability (life).

The catalyst of the present invention is defined in appended claim 1.

The detailed description which follows will serve to make the structure and advantages of the catalyst according to this invention more clearly understood.

In the description, the expression "potentiality of the catalyst" refers to the amount of raw material which can be converted by contacting the catalyst per unit volume of catalyst in the reactor.

Thus, the catalyst for the preparation of maleic anhydride comprises, in accordance with this invention, a support or carrier made up of open arcuate shape particles. The arc defined by the support particle may be an arc of a circle, or of an ellipse, or parabola, etc., thereby said arc may have different radius portions. Preferably, the arc defined by the support particles is an arc of a circle, and the preferred support particles are those in the form of half rings although other forms may be utilised in the present invention. Accordingly, in the following description, specific reference will be made to the half ring forms of the support.

Half rings are preferably utilized which have outside diameters in the 4 to 10 mm range, inside diameters in the 1 to 8 mm range, and lengths i.e. axial dimensions in the 3 to 8 mm range. As an example, a preferred half ring configuration has, in accordance with the invention, an outside diameter of 7 mm, an inside diameter of 4 mm, and an axial dimension of 5 mm.

The half ring support of the catalyst of this invention has a geometric specific surface area (geometric surface area per reactor unit volume) which is higher than that of conventional supports or carriers for oxidizing benzene to maleic anhydride, affording a considerable improvement of the catalyst overall performance. In Table 1 hereinbelow, comparative data are given for the geometric surface areas provided by catalyst supports in the form of balls of various diameters, cylinders, and ring or half ring supports according to the invention.

2

TABLE 1

| Carrier (dimensions in mm) | Total geometric surface area in the reaction tube[+] ($m^2$) |
|---|---|
| Rings, 5 × 2 × 5[++] | 1.13 |
| Half rings, 7 × 4 × 5[++] | 1.17 |
| Cylinders, 5 × 5 | .93 |
| Balls, Dia. = 5 | .78 |

[+] Dia. = 21 mm; h = 3,200 mm
[++] O.D.×I.D. × Length

This increased geometric surface area affords higher potentiality, selectivity, and durability or activity of the catalyst, with all other performances being equivalent, with respect to the cited conventional catalysts or supports.

Thus, for the same selectivity, it becomes possible to achieve a higher potentiality, in that one can have a larger amount of active mass in the reactor, correspondingly to the increased surface area of the support, without increasing the thickness or depth of the catalytic layer. Thus, a higher flow rate of reactant to be treated (e.g. benzene) can be achieved for a given thickness of the catalytic layer in the reactor. Table 2 hereinbelow, illustrates in fact a comparison of the potentialities of conventional catalysts using known supports, and of a catalyst according to this invention, said potentiality being expressed in terms of benzene mass flow rate versus the same thickness or depth of the catalytic layer.

TABLE 2

| Support (dimensions in mm) | Total geometric surface area in the reaction tube ($m^2$) | Benzene mass flow rate (g/hour) |
|---|---|---|
| Rings, 5 × 2 × 5 | 1.13 | 146 |
| Half rings, 7 × 4 × 5 | 1.17 | 151 |
| Cylinders, 5 × 5 | .93 | 120 |
| Balls, Dia. = 5 | .78 | 101 |

By converse, by maintaining a constant reactant feed at the start, relatively to other supports, it is possible to achieve, with the inventive catalysts, improved selectivities thankes to the possibility of having the same total amount of active catalytic mass in the tube, with a lower thickness of the catalytic layer, and accordingly less over-oxidation phenomena; actually, it is a well known fact that the thickness of the catalytic layer in a reactor is to reconcile conflicting requirements, since on one side, an increased thickness affords a larger active catalytic mass and a higher conversion output, whilst on the other side, a high thickness may result in overoxidation phenomena and the production of undesired byproducts. Thus, it is of importance that a catalyst should present a larger active mass distributed over a thinner catalytic layer, which is indeed accomplished by the catalyst according to this invention.

Of course, the catalyst of this invention ensures a simultaneous increase of both the potentiality and selectivity of the catalyst with a smaller thickness catalytic layer with respect to conventional supports and a slightly lower feed than shown in Table 2, but still much higher than is afforded by other conventional supports.

A smaller thickness or depth of the active portion results in a temperature pattern through the catalytic bed, for a given set of conditions, which is flatter than in conventional catalysts. Thus for example, for the same feed, the catalyst of this invention enables one to operate at lower temperatures, and temperature peaks can be avoided in the catalytic mass which would affect the desired product yield.

By virtue of said flatter temperature pattern, and accordingly reduced thermal stresses, it also becomes possible to operate at higher concentrations of benzene in air, and to achieve a longer life or durability of the catalyst activity than in conventional catalysts.

For manufacturing the supports or carriers of the catalysts according to this invention, any inert material may be utilized which is normally used in the manufacture of catalyst supports, such as alumina, with the addition of minor amounts of silica and oxides of alkaline and alkaline-earth metals, or silicates (e.g. steatite). An exemplary composition of a support or carrier useful in implementing this invention is the following:

| | |
|---|---|
| $Al_2O_3$ | 87.02 percent |
| $SiO_2$ | 9.63 |
| $TiO_2$ | .85 |
| $CaO$ | 1.38 |
| $Fe_2O_3$ | .52 |
| $MgO$ | .12 |
| $Na_2O$ | .21 |
| $K_2O$ | .27 |

The method for preparing the catalyst is characterized in that it comprises the following steps: a porous support is impregnated with a solution of the active catalyst components, then dried for 7 to 8 hours at a temperature below 100°C, and finally calcined at a temperature above 400°C for 3 to 7 hours in an enclosed environment.

The active mass of the catalyst for the preparation of maleic anhydride, to be used in conjunction with the catalytic support according to the invention preferably comprises, as its basic components, molybdenum oxide and vanadium oxide, with the addition of various auxiliary components having promoting, stabilizing, and other functions. The composition of the active mass of the catalyst, as expressed in terms of parts by weight of oxides in the starting materials for the preparation of the catalyst, may vary within the following ranges: $MoO_3$, 1; $V_2O_5$, 1.2—2.5; $Bi_2O_3$, 0.02—0.64; $P_2O_5$, 0.02—0.05; $Na_2O$, 0.03—0.06; $NiO$, 0.03—0.06.

An exemplary composition may be the following: 1 part $MoO_3$; 2.1 parts $V_2O_5$; 0.03 parts $P_2O_5$; 0.04 parts $Na_2O$; 0.04 parts $NiO$; and 0.06 parts $Bi_2O_3$.

The advantages to be secured with the catalysts of this invention will be illustrated by the examples which follow, by way of illustration only and not of limitation of the true scope of the invention.

### Example 1: Preparation of the Catalysts

In a suitable vessel of an acid-resistant material, there are introduced 71.43 parts (by weight) of 33% concentrated hydrochloric acid, 6.07 parts of ammonium paramolybdate, and 13.22 parts of ammonium metavanadate, which are then stirred for a few minutes.

The following solutions are prepared in suitable vessels: in a first vessel, a solution of .79 parts trisodium phosphate (dodecahydrate) in 3.59 parts water; in a second vessel, a solution of .64 parts bismuth nitrate (pentahydrate) in 1.28 parts water; and in a third vessel, .71 parts nickel nitrate (hexahydrate) in 2.27 parts $H_2O$.

These three solutions are then added to the hydrochloric solution prepared in the first-mentioned vessel, and the whole is stirred occasionally until the time of using it.

With this solution, some supports are impregnated which have the same chemical composition but different shapes and dimensions. One operates with different amounts of the solution for each support, taking into account the different specific geometric surface areas of the supports, so as to maintain the thickness or depth of the active catalytic layer on the carriers practically at the same value.

The coating operation is carried out by slow evaporation of the solution in accordance with conventional practices. Finally, the catalyst is calcined at about 400°C for 4 hours in an enclosed environment. The characteristics of the resulting catalysts are indicated in the following table.

## TABLE 3

| Catalyst identification | Support shape and dimensions (mm) | Solution added g solution/ 100 g support | Final percent active components g/100 g support (after calcination) |
|---|---|---|---|
| A | Rings 5 × 2 × 5* | 125 | 188 |
| B | Half rings 7 × 4 × 5* | 128.5 | 18.5 |
| C | Cylinders 5 × 5 | 102.8 | 14.8 |
| D | Balls ∅ = 5 | 86.8 | 12.5 |

\* = outside ∅ × inside ∅ × axial dimension

Example 2: Catalytic Performance

Catalysts A, B, C and D of Table 3 were loaded to a height level of 320 cm into a reactor comprising a tube having and inside diameter of 21 mm, and being immersed in a molten salt bath and provided on its inside with a sleeve having a diameter of 3 mm, for a movable thermocouple (adapted for controlling the internal temperature of the catalytic bed).

With each catalyst, tests have been carried out in three different conditions of feed.

The conditions and results are indicated in Table 4.

TABLE 4

| Catalyst | Space velocity $(h^{-1})$ | Benzene mass flow rate (g/hour) | Benzene concentration (moles %) | Salt Temperature (°C) | Hot spot (°C) | Catalyst performance | | | |
|---|---|---|---|---|---|---|---|---|---|
| | | | | | | After 30 days | | After 180 days | |
| | | | | | | Benzene conversion (%) | Maleic anhydride yield (g%) | Benzene conversion (%) | Maleic anhydride yield (g%) |
| A | 2800 | 120 | 1.23 | 355 | 403 | 98 | 96 | 98 | 95.5 |
| A | 2800 | 150 | 1.52 | 365 | 430 | 97 | 95 | 97.5 | 95 |
| A | 2325 | 150 | 1.81 | 365 | 450 | 97 | 94 | 97 | 94 |
| B | 2800 | 120 | 1.23 | 355 | 405 | 98 | 96 | 98 | 96 |
| B | 2800 | 150 | 1.52 | 360 | 435 | 97 | 94 | 98 | 95 |
| B | 2325 | 150 | 1.81 | 365 | 455 | 97 | 94 | 97 | 94.5 |
| C | 2800 | 120 | 1.23 | 360 | 420 | 97 | 94 | 96 | 92 |
| C | 2800 | 150 | 1.52 | 370 | 450 | 96 | 92 | 95 | 90 |
| C | 2325 | 150 | 1.81 | 370 | 465 | 95 | 88 | 93 | 85 |
| D | 2800 | 120 | 1.23 | 360 | 415 | 97 | 93 | 96 | 91 |
| D | 2800 | 150 | 1.52 | 370 | 450 | 95 | 90 | 93 | 87 |
| D | 2325 | 180 | 1.81 | 380 | 470 | 94 | 87 | 90 | 82 |

0 037 020

Catalysts A and B already give better results at low benzene mass flow rates; however, the results become definitely better than with catalysts based on conventional supports (C and D) by operating with higher mass flow rates and at higher concentrations of benzene in air.

At the end of 180 days, the yield in maleic anhydride and the benzene conversion rate are slightly increased for catalyst B according to this invention, even in conditions of maximum feed and concentration, whereas for catalysts C and D a marked drop of the values can be observed in the maximum feed conditions, as due to deactivation of the catalysts.

**Claims for the Contracting States: CH LI FR DE GB**

1. A catalyst for use in the preparation of maleic anhydride by oxidation of benzene with an oxygen containing gas, in which the main components of the catalyst are $MoO_3$, $V_2O_5$, $Bi_2O_3$, $P_2O_5$ and $Na_2O$, $SiO_2$ characterized in that it comprises a porous support or carrier having particles in the form of half-rings; and an active portion distributed over said porous alumina support or carrier, said active portion comprising 1 part $MoO_3$; 1.2 to 2.5 parts $V_2O_5$; 0.02 to 0.64 parts $Bi_2O_3$; 0.02 to 0.05 parts $P_2O_5$; 0.03 to 0.06 parts $Na_2O$; and 0.03 to 0.06 parts NiO.

2. The catalyst according to claim 1, characterized in that the particles in the form of half-rings have outside diameters in the 4 to 10 mm range, inside diameters in the 1 to 8 mm range; and an axial dimension in the 3 to 8 mm range.

3. The catalyst according to claim 1, characterized in that the active portion distributed over the porous support or carrier comprises 1 part $MoO_3$; 2.1 parts $V_2O_5$; 0.03 parts $P_2O_5$; 0.04 parts $Na_2O$; 0.04 parts NiO and 0.06 parts $Bi_2O_3$.

4. The catalyst according to claim 1, characterized in that said support or carrier is chosen between alumina and silicates.

**Claims for the Contracting State: AT**

1. A process for the preparation of maleic anhydride by oxidation of benzene with an oxygen containing gas, in which a catalyst is used the main components of which are $MoO_3$, $V_2O_5$, $Bi_2O_3$, $P_2O_5$ and $Na_2O$, $SiO_2$ characterized in that the catalyst used comprises a porous support or carrier having particles in the form of half-rings; and an active portion distributed over said porous alumina support or carrier, said active portion comprising 1 part $MoO_3$; 1.2 to 2.5 parts $V_2O_5$; 0.02 to 0.64 parts $Bi_2O_3$; 0.02 to 0.05 parts $P_2O_5$; 0.03 to 0.06 parts $Na_2O$; and 0.03 to 0.06 parts NiO.

2. A process according to claim 1, characterized in that the catalyst support particles in the form of half-rings have outside diameters in the 4 to 10 mm range, inside diameters in the 1 to 8 mm range; and an axial dimension in the 3 to 8 mm range.

3. A process according to claim 1, characterized in that the active portion of the catalyst distributed over the porous support or carrier comprises 1 part $MoO_3$; 2.1 parts $V_2O_5$; 0.03 parts $P_2O_5$; 0.04 parts $Na_2O$; 0.04 parts NiO and 0.06 parts $Bi_2O_3$.

4. A process according to claims 1—3, characterized in that said support or carrier of the catalyst is chosen between alumina and silicates.

**Patentansprüche für die Vertragsstaaten: CH LI FR DE GB**

1. Katalysator zur Anwendung in der Herstellung von Maleinsäureandhydrid durch Oxidation von Benzol mit einem sauerstoffhaltigen Gas, wobei die Hauptkomponenten das Katalysators $MoO_3$, $V_2O_5$, $Bi_2O_3$, $P_2O_5$, $Na_2O$ und $SiO_2$ sind, dadurch gekennzeichnet, dass er aus einem porösen Träger aus Teilchen in Form von Halbringen und einem auf dem genannten porösen Tonerdeträger verteilten aktiven Anteil besteht, der aus 1 Teil $MoO_3$; 1,2 bis 2,5 Teilen $V_2O_5$; 0,02 bis 0,64 Teilen $Bi_2O_3$; 0.02 bis 0,05 Teilen $P_2O_5$; 0,03 bis 0,06 Teilen $Na_2O$; und 0,03 bis 0,06 Teilen NiO besteht.

2. Katalysator nach Anspruch 1, dadurch gekennzeichnet, dass die halbringförmigen Teilchen Aussendurchmesser im Bereich von 4 bis 10 mm, Innendurchmesser im Bereich von 1 bis 8 mm und eine Axialabmessung im Bereich von 3 bis 8 mm besitzen.

3. Katalysator nach Anspruch 1, dadurch gekennzeichnet, dass der auf dem porösen Träger verteilte aktive Anteil aus 1 Teil $MoO_3$; 2,1 Teilen $V_2O_5$; 0,03 Teilen $P_2O_5$; 0,04 Teilen $Na_2O$; 0,04 Teilen NiO und 0,06 Teilen $Bi_2O_3$ besteht.

4. Katalysator nach Anspruch 1, dadurch gekennzeichnet, dass der Träger aus der Gruppe Tonerde und Silikate ausgewählt ist. ·

**Patentansprüche für d ịn Vertragsstaat: AT**

1. Verfahren zur Herstellung von Maleinsäureanhydrid durch Oxidation von Benzol mit einem sauerstoffhaltigen Gas unter Verwendung eines Katalysators, dessen Hauptkomponenten $MoO_3$, $V_2O_5$, $Bi_2O_3$, $P_2O_5$, $Na_2O$ und $SiO_2$ sind, dadurch gekennzeichnet, daß ein Katalysator eingesetzt wird, der aus einem porösen Träger aus Teilchen in Form von Halbringen und einem auf dem genannten porösen Tonerdeträger verteilten aktiven Anteil besteht, der aus 1 Teil $MoO_3$; 1,2 bis 2,5 Teilen $V_2O_5$; 0,02 bis 0,64 Teilen $Bi_2O_3$; 0.02 bis 0,05 Teilen $P_2O_5$; 0,03 bis 0,06 Teilen $Na_2O$; und 0,03 bis 0,06 Teilen NiO besteht.

2. Verfahren nach Anspruch 1, dadurch gekennzeichnet, daß ein Katalysator eingesetzt wird, dessen halbringförmige Trägerteilchen Außendurchmesser im Bereich von 4 bis 10 mm, Innendurchmesser im Bereich von 1 bis 8 mm und eine Axialabmessung im Bereich von 3 bis 8 mm besitzen.

3. Verfahren nach Anspruch 1, dadurch gekennzeichnet, daß ein Katalysator eingesetzt wird, dessen auf dem porösen Träger verteilter aktiver Anteil aus 1 Teil $MoO_3$; 2,1 Teilen $V_2O_5$; 0,03 Teilen $P_2O_5$; 0,04 Teilen $Na_2O$; 0,04 Teilen NiO und 0,06 Teilen $Bi_2O_3$ besteht.

4. Verfahren nach den Ansprüchen 1 bis 3, dadurch gekennzeichnet, daß ein Katalysator eingesetzt wird, dessen Träger aus der Gruppe Tonerde und Silikate ausgewählt ist.

**Revendications pour les Etats contractants: CH LI FR DE GB**

1. Un catalyseur pour la préparation d'anhydride maléique par oxydation de benzene avec un gaz contenant de l'oxygene dans lequel les constituants principaux du catalyseur sont $MoO_3$, $V_2O_5$, $Bi_2O_3$, $P_2O_5$, et $Na_2O$, $SiO_2$, caractérisé par le fait qu'il comprend un support poreux ayant des particules en forme de demi-anneaux; et une partie active répartie sur ledit support poreux en alumine, ladite partie active comprenant 1 partie de $MoO_3$; 1,2 à 2,5 parties de $V_2O_5$; 0,02 à 0,64 parties de $Bi_2O_3$; 0,02 à 0,05 parties de $P_2O_5$; 0,03 à 0,06 parties de $Na_2O$; et 0,03 à 0,06 parties de NiO.

2. Le catalyseur selon la revendication 1, caractérisé par le fait que les particules en forme de demi-anneaux ont des diamètres extérieurs dans la gamme de 4 à 10 mm, des diamètres intérieurs dans la gamme de 1 à 8 mm; et une dimension axiale dans la gamme de 3 à 8 mm.

3. Le catalyseur selon la revendication 1, caractérisé par le fait que la partie active du catalyseur répartie sue le support poreux comprend 1 partie de $MoO_3$; 2,1 parties de $V_2O_5$; 0,03 parties de $P_2O_5$; 0,04 parties de $Na_2O$; 0,04 parties de NiO et 0,06 parties de $Bi_2O_3$.

4. Le catalyseur selon la revendication 1, caractérisé par le fait que ledit support est choisi parmi l'alumine et les silicates.

**Revendications pour l'Etat contractant: AT**

1. Procédé pour la préparation d'anhydride maléique par oxydation de benzene avec un gaz contenant de l'oxygene dans lequel est utilisé un catalyseur dont les constituants principaux sont $MoO_3$, $V_2O_5$, $Bi_2O_3$, $P_2O_5$, et $Na_2O$, $SiO_2$, caractérisé par le fait que le catalyseur utilisé comprend un support poreux ayant des particules en forme de demi-anneaux; et une partie active répartie sur ledit support poreux en alumine, ladite partie active comprenant 1 partie de $MoO_3$; 1,2 à 2,5 parties de $V_2O_5$; 0,02 à 0,64 parties de $Bi_2O_3$; 0,02 à 0,05 parties de $P_2O_5$; 0,03 à 0,06 parties de $Na_2O$; et 0,03 à 0,06 parties de NiO.

2. Le procédé selon la revendication 1, caractérisé par le fait que les particules du support du catalyseur en forme de demi-anneaux ont des diamètres extérieurs dans la gamme de 4 à 10 mm, des diamètres intérieurs dans la gamme de 1 à 8 mm; et une dimension axiale dans la gamme de 3 à 8 mm.

3. Le procédé selon la revendication 1, caractérisé par le fait que la partie active du catalyseur répartie sur le support poreux comprend 1 partie de $MoO_3$; 2,1 parties de $V_2O_5$; 0,03 parties de $P_2O_5$; 0,04 parties de $Na_2O$; 0,04 parties de NiO et 0,06 parties de $Bi_2O_3$.

4. Le procédé selon les revendications 1—3, caractérisé par le fait que ledit support du catalyseur est choisi parmi l'alumine et les silicates.